# EUROPEAN PATENT APPLICATION

(11) **EP 0 721 000 A2**
(43) Date of publication of application: **10.07.1996**
(21) Application number: 96500003.7
(22) Date of filing: 09.01.1996
(51) Int. Cl.: C08J 3/24, C08L 95/00

(54) **Process for the preparation of cross-linked hydrocarbonated-polymeric binders**

(30) Priority: 09.01.1995 ES 9500017
(71) Applicant: REPSOL PETROLEO S.A., E-28046 Madrid (ES)
(72) Inventor: Sortano, Jose Maria Molina, E-30202 Cartagena (ES); Hernandez, Andres Hernandez, E-30009 Murcia (ES); Rodriguez, Francisco Domingo, E-28019 Madrid (ES)
(74) Representative: Garcia Cabrerizo, Francisco

(57) **Abstract**

The process basically consists in initially preparing a polymeric concentrate; the polymer, a thermoplastic elastomer of the styrene-butadiene type and with adequate Mooney viscosity, is dissolved, by means of stirring and temperature, in a petroleum derived solvent which ends up forming part of the final product. The homogeneity of this concentrate is controlled "in situ" by electronic monitoring of the viscosity, a fundamental property.

Later, the polymeric concentrate is made to react isothermically and under stirring with an asphaltene concentrate in the presence of a cross-linking system of the kind used in the vulcanization of rubber. The termination of the reaction is controlled "in situ" by electronic measurement of the viscosity, a fundamental property.

The isothermicity of the whole operation allows the process to be simple and cost-effective.

The product obtained, a fluid cross-linked hydrocarbonated polymeric binder, finds application in the preparation of asphaltic agglomerates and as sealant in construction joints.

## Description

The present invention defines a process for the preparation of cross-linked hydrocarbonated-polymeric binders, which can be generally used in the making of bituminous mixtures, and more specifically, in the surfacing of roads.

The use of hot bituminous mixtures as covering for diverse surfaces and in particular as wearing courses, is adequate as long as these compositions have a number of essential mechanical properties.

These mechanical properties are appreciated in practice by determining, by means of standardised assays, a number of characteristics the values of which have to comply with certain predetermined specifications. These characteristics, of physical and elastomeric nature, which define the specific application of the product obtained, are basically penetration, viscosity, brittlenes, low temperature ductility, elastic recovery and tensile stress, among others.

In general, it is known that conventional bitumens, obtained from the heavy fractions of petroleum distillation, do not simultaneously exhibit the full set of characteristics currently demanded for their use as binders in mixtures with arids; it is also long known that the addition of certain types of polymers to these improve their mechanical properties. This is of vital importance when the intended use is in road surfacing , where currently both the number of vehicles and the weight transported per axle has experienced a great increase.

The polymers susceptible of being added are mainly elastomers, like for example : rubber-butyl, polybutyl, EVA copolymers, terpolymers like ethylene/propylene/diene conjugates (EPDM), as well as the well known statistical or sequential copolymers of styrene and a conjugated diene (thermoplastic elastomers).

Industrial interest has focused on these copolymers, both for their ease in dissolving in the heavier petroleum fractions as for conferring excellent mechanical-dynamic properties to the mixture obtained, and more specifically, good viscoelastic properties. This translates into a product with a greater wrapping capacity in the step involving the mixing with arids, a greater adhesion to these and more independence in its behaviour in relation to external agents such as ambient temperature and load.

It is known that the storage stability of these hydrocarbonated polymeric mixtures can be improved, in relation to simple physical mixing, by chemical coupling or chemical cross-linking, since a composition which is totally homogeneous and indivisible with time is obtained.

The methods for the preparation of this last type of compositions as well as their characteristics are described in the literature and within patents for industrial exploitation. Two essential types of preparation are worth remarking :
- Direct solution of the polymer in a heavy fraction of petroleum with a high asphaltene content, and subsequent reaction in an appropriate medium.
- Preparation of a polymeric concentrate and subsequent addition and reaction with an asphaltene concentrate.

It is also worth noting that during the reaction step, similarly to what happens during the vulcanization of rubber, the addition of compounds known as "vulcanization accelerators" improve the coupling step of the vulcanizing agent, generally sulphur (which may or may not form part of the molecule of such accelerator), and therefore, the characteristics of the final product.

The reaction mechanism which takes place between the sulfur, the asphaltenes and the styrene-diene conjugated copolymer is not perfectly defined; notwithstanding, one may estimate it is quite analogous to that occuring in the vulcanization process of rubber. The existing sulphur produces, in an adequate reaction medium, a modification in the structure of the original mixture, composed mainly by asphaltenes, giving rise to hydrocarbonated polymeric binders with mechanical properties and thermal stabilities superior to those of conventional bitumens.

In general it can be said that the added sulphur acts both as polymer-asphaltene coupling agent and as a cross-linking agent between the polymer chains (cross-linked effect).

It is important to ensure the homogeneity of the mixture between the polymeric concentrate and the asphaltene-rich fraction prior to the addition of the accelerating/cross-linking complex, as premature addition could lead to bulk solidification (cross-linkage of the polymer with itself), as a consequence of the ultra-rapid vulcanization of the components in the mixture, producing a heterogeneous cross-linked mixture which consequently is of no use for the purpose intended.

The mechanical-elastical characteristics of the products obtained, determined by the traction assays, are in comparison to conventional bitumens, improved at low temperatures and are mostly maintained after being subjected to simulated aging assays.

This conservation of characteristics allows that in practice such products may be prepared by manufacturing campaigns and stored at sufficiently high temperatures, in the range of 120 - 180 °C, to maintain the required fluidity until their utilization.

The patent of invention Nr. P9202344, describes and claims a process for the preparation of complex hydrocarbonated polymeric cross-linked compositions, characterized in that, in a first stage, a "preactivated" polymeric concentrate is prepared by the mixing of a hydrocarbonated solvent, a styrene-butadiene random copolymer of an average molecular weight above 300.000 and of a mixture of fatty acids or of their salts. This concentrate is mixed with an asphaltene concentrate for its subsequent cross-linking, within the same reactor, by means of a similar cross-linkage system as the one used in the vulcanization of rubber.

The resulting composition can be employed as a binder in the elaboration of asphaltic agglomerates.

The type of polymer used in said application is a random styrene-butadiene which, due to its special configuration, requires a preactivation step to facilitate the cross-linking process.

The present invention has the object of extending the process to the preparation of hydrocarbonated polymeric cross-linked binders where the copolymers are chosen so as to make it unnecesary to have a preactivation step. Also, in order to improve the process, isothermal operational conditions and electronic control of the production stages are selected which determine, in a direct manner at each stage, the values of an essential property which defines the products obtained : viscosity. This set of actions permit both an optimization of the process and a reduction in the total production costs.

The process related to the present invention fundamentally consists in chemically bonding certain potentially active centers of the polymer molecule with certain hydrocarbonated molecules derived from petroleum which have the appropriate reactivity. The final product becomes fully integrated within the hydrocarbonated matrix forming just one phase which remains stable with time (constant homogeneity, oxidation state and viscosity).

In a similar manner to the well known process of rubber vulcanization, the process herein allows the obtention of hydrocarbonated polymeric binders with varying degrees of cross-linking, depending upon the working conditions and the type of polymer used for a given cross-linkage system. These compositions behave in a similar manner to bitumens modified by physical mixing of the bitumen and the polymer, without having the aforementioned stability problems.

In the first stage of the process, a concentrate of the polymer is prepared as a result of which it is dissolved and left ready for the cross-linking reaction or second stage. This takes place by the mixing of the polymer concentrate with a petroleum fraction with a high asphaltene content, at the same temperature as the first stage, in the presence of a metallic catalyst and an accelarator/cross-linking system of the kind employed in the vulcanization of rubber. Sulphur is used as cross-linking agent, which can in turn be provided to the medium or generated "in situ" by the accelerator if it itself is at the same time a sulphur donor as in the case, for instance, of 2-benzothiazol dithio-morpholine, dibutyl zinc dithiophosphate and of some polyalkyl thiourames polysulfides among others.

It is also known that the absence of elemental sulphur in the medium results, as most significant feature, in that the composition of the vulcanization obtained is richer in monosulfide bridges; stronger bonds than the polysulfide bridges formed between elastomer chains in the presence of elemental sulphur.

The choice of one or another acceleration system will have an effect in the sense that the monosulfide to polysulfide bond ratio (and on the occasional formation of carbon-carbon bonds) in the cross-linking process will be different, hence its influence upon the elastic properties of the final product.

These properties will also be dependent upon the type and relative concentrations of the components selected for the formulation of the final product.

The copolymers chosen in the preparation process object of the present patent are of the SBR family and of the gradual block or even block/random type, with lineal and radial structures and with no limitations on their molecular weights. Nevertheless, the copolymer may include other types of conjugated dienic groups such as isoprene.

An essential characteristic to be considered during polymer selection is its Mooney viscosity value, which must be such as to provide the final product obtained with sufficient fluidity for its direct use in the mixing with arids at normal field working temperatures, as well as for its direct application in other industrial areas such as production of sealants and insulation linings.

A heavy fraction of petroleum distillation, rich in asphaltenes, is used as source asphaltenes, the major characteristics of which are :
- Asphaltene content above 7% as determined by the method IP-143.
- Flashpoint above 235 °C, measured following ASTM standard assay D-92/90.

The solvent employed, which is part of the final product even after its utilization as binder in asphaltic agglomerates or in construction joints, is a hydrocarbonated compound characterized by possesing an aniline point with values ranging from 60 to 80 °C as determined according to ASTM standard D-611, and a kinematic viscosity at 100 °C ranging between 40 cSt and 70 cSt (ASTM-D-445).

From the experimental point of view, a way of carrying out the preparation process of cross-linked hydrocarbonated polymeric binders consists in mixing, under agitation and at a constant temperature between 120 and 180 °C, all the ingredients which form part of the preparation process of the polymeric concentrate; these are : the hydrocarbonated solvent, which is additioned in such a way as to ensure the total solubility of the polymer, and the polymer, calculated so that its final concentration in the final product ranges between 2 and 6 parts per hundred (pph). The mixing time will depend on the type of polymer employed, thus it is essential to use an electronic system to measure "in situ" the homogeneity of the polymeric concentrate by determining the viscosity as a basic property. This has to be found between 400 and 800 cSt (at the reaction temperature) if the final product to be obtained is to be used as a binder, both in the formulation of bituminous mixtures as in construction waterproofing.

Once the polymeric concentrate has been obtained, it is mixed under agitation with the asphaltene rich petroleum fraction, both at the same temperature, which coincides with that of the first stage.

The relative ratio of both within the mixture will be a function of the specifications required from the final product to be obtained, which will in turn depend on the type of application to which it will be subjected.

This, however, is not absolutely critical, since it is possible to adjust to some of the required specifications by adding more solvent to the final product, or even by the use of oxidation techniques of the kind generally employed in the oxidized bitumen industry.

After a mixing period not below 15 minutes, the metallic catalyst, the cross-linking agent and the vulcanization accelerator are added, or otherwise just the metallic catalyst and the vulcanization accelerator when it itself generates the cross-linking agent "in situ". The reaction step is isothermic, coinciding in temperature with that of the polymeric concentrate preparation stage.

To ensure optimum cross-linking, the amount of sulphur in the reaction medium must not be below 0.5 pph, and shall preferrably be between 1 and 3 pph.

The amount of metallic catalyst shall be comprised between 0.2 and 2 pph.

The necessary amount of vulcanization accelerator will depend both upon the capacity of the medium to provide sulphur to the reaction and on the type of system employed (mono, binary, etc.). An appropriate system shall be that in which the total concentration ranges between 1.5 and 5 pph, considering that, if it is also used as a sulphur donor, the concentration of the latter in the reaction medium shall not fall below 0.5 pph.

In the present invention, combined accelerator/cross-linking combination systems of the kind employed in the rubber industry, and which in addition produce rapid vulcanizations with no risks of prevulcanization, have been selected as accelerators.

This choice has been made in order to achieve "non sulfur" curates of totally different characteristics to those produced when free sulphur is added to the medium; here, the curing process is slower, there is greater safety at the overgaging step and a greater "plateau" effect is obtained, resulting in products exhibiting an excellent resistance to thermal aging as well as to reversion (loss of properties due to overgaging).

Control over the reaction is critical, this being performed by means of an electronic system which, by measuring an essential property, indicates how and when the reaction has terminated. As in the first stage, vlscosity is such property. The value of this property shall be comprised between 500 and 900 cSt (at the reaction temperature) if the final product to be obtained is to be used as a binder, both in the formulation of bituminous mixtures as in construction waterproofing.

The required properties are measured on the product obtained, and if necessary, these are adjusted with the same solvent used in the preparation of the polymeric concentrate or by means of the aforementioned oxidation techniques.

The products obtained by the process according to the present invention exhibit a strongly elastic character, a great plasticity range, which makes them particularly interesting for the production of various coatings, and in particular, for the surfacing of roads. For this fundamental application, the mixtures of the present invention which are elaborated by the preparation of a polymeric concentrate technique, are particularly suitable as they can be employed directly by the classical means of mixing with arids and extension.

The present invention is illustrated by means of the following examples given on a non-limiting basis, considering the diversity of useful copolymers for a particular cross-linking system.

A blank assay has also been performed (in the absence of cross-linking agent) as shown in example A.

The rheological and mechanical characteristics of bitumens or of hydrocarbonated polymeric binders referred to in these examples are the following :
- Penetration. Expressed in 1/10ths of a mm according to standard ASTM-D-5-73/78 (IP-49/76 or NLT 124/84).
- Fraass point or cold brittlenes point, expressed in °C and determined according to method IP-80/53 (DIN 52012/80 or NLT 182/84).
- Kinematic viscosity. Expressed in centistokes, determined according to ASTM standard D-445.
- Ductility at 5 °C. Expressed in cms. and determined according to standard ASTM-D-113/79 (NLT 126/84).
- Elastic recovery in a ductilometer at 10 °C and expressed as a percentage (%).
- Tensile rheological characteristics determined in a ductilometer, at 10 °C, with a spatial charge cell and an elongation velocity of 50 mm/m. The parameters evaluated are :
- Rupture elongation (Er) in %.
- Rupture elongation stress (*ó*ᵣ) in kg/cm²).
- Stress at 1.000 % elongation.
- Stress at 2.000 % elongation.
- Maximum threshold stress (bitumen RM) in kg/cm².
- Maximum stress due to the polymer (polymer RM) in kg/cm².
- RMP/RMB ratio.
- TFOT assay according to standard ANSI/ASTM-D-1754/78 (NLT-185).

### EXAMPLE A

The polymeric concentrate referred to in the first stage has been prepared mixing at 150 °C under agitation, 30 parts of hydrocarbonated solvent with 4 parts of polymer, until total homogeneity is obtained, as evaluated dynamically "in situ" by means of its viscosity. The product obtained has a kinematic viscosity of 600 cSt at 135 °C.

The hydrocarbonated solvent exhibits an aniline point between 65 and 75 °C, and a kinematic viscosity in the range of 55 - 65 cSt at 100 °C. The commertial polymer used belongs to the styrene-butadiene family, it is polymerysed in a gradual block and has a Mooney viscosity (ML4 at 100 °C) - below 50.

This polymeric concentrate is mixed, under stirring and at a temperature of 150 °C, with 100 parts of an asphaltene concentrate, characterized by having a flashpoint of over 235 °C and an asphaltene content above 10%.

### EXAMPLE B

The polymeric concentrate was prepared in exactly the same manner as in example A. Said concentrate was mixed over 15 minutes with 100 parts of an asphaltene concentrate identical to that employed in example A. Subsequently, under stirring at 150 °C, 0.7 parts of zinc oxide and 2 parts of tetramethylon thiouramium disulfide where added.

The reaction was considered terminated when the viscosity of the medium reached a constant value of 575 cSt at the reaction temperature.

### EXAMPLE C

The whole of the operation is identical to that in example B, save for changing the polymer for another of the styrene-butadiene type with random-block configuration and linear structure and with a Mooney viscosity of 60. The duration of the first stage is under 6 hours and that of the second stage is the same as in example B.

### EXAMPLE D

Identical to C but the polymer used, similar to the previous one but with a star like structure, has a Mooney viscosity, before coupling, of 63. The duration of the first stage is 5 hours and that of the second stage is the same as in example B.

### EXAMPLE E

Identical to example B, it serves as a base for the preparation of example F. In this case, 32 parts of hydrocarbonated solvent, 0.5 parts of catalyst and 1.5 parts of cross-linking agent are used.

### EXAMPLE F

The product was obtained by controlled (in relation to flow, time and temperature) oxidation with air of the product obtained in E.

### EXAMPLES G and H

These are the products resulting from the TFOT aging assays of products E and F respectively.

Table I shows the characteristics of the cross-linked hydrocarbonated polymeric binders obtained, with physical and elastomeric characteristics similar to those of polymer modified bitumens, which permits their use for the same purposes.

Table II shows the influence which the oxidation of a product has over the obtention of another one, without any loss in elastic properties, but which also complies with other types of required specifications.

Here one may also observe the characteristics of both after being subjected to a simulated aging experiment known as "TFOT" or residue after fine film oxidation.

Having sufficiently described the nature of the object of the invention, as well as the way for its practical realization, it must be noted that stipulations previously stated are liable of being modified in the detail as long as the fundamental principle remains unaffected.

**TABLE I**

| **PROPERTY** | | **A** | **B** | **C** | **D** |
|---|---|---|---|---|---|
| PHYSICAL PROPS. | | | | | |
| Penet. 25 °C | 0.1 mm | 72 | 61,0 | 59,2 | 64,2 |
| Frass | °C | - | -16 | -16 | -20 |
| Viscosity 135 °C | cst | 926 | 1340 | 1756 | 1159 |
| Viscosity 165 °C | cst | 242 | 330 | 417 | 291 |

| ELASTOM. PROPS. | | | | | |
|---|---|---|---|---|---|
| Ductility 5°C | cm | 4 | 21 | 13 | 21 |
| Elast.recov. 10 °C | % | R-9 | 70 | 69,5 | 69 |
| Def. E = 1000 % | Kg/cm² | - | 1,58 | 1,40 | 1,33 |
| Def. E = 2000 % | Kg/cm² | - | 1,70 | 1,78 | 1,23 |
| RM Bitumen | Kg/cm² | 3,28 | 3,66 | 3,17 | 2,93 |
| RM Polymer | Kg/cm² | - | 1,73 | 1,79 | 1,34 |
| Rupture elong. | E % | 700 | 3300 | 2800 | 3300 |
| | Kg/cm² | 0 | 1,54 | 1,56 | 0,33 |
| RPM / RMB | | - | 0,47 | 0,56 | 0,46 |

**TABLE II**

| **PROPERTY** | | **E** | **F** | **G** | **H** |
|---|---|---|---|---|---|
| PHYSICAL PROPS. | | | | | |
| Penet. 25 °C | 0.1 mm | 70,4 | 56,6 | 46,9 | 45,5 |
| Frass | °C | -18 | -20 | - | - |
| Viscosity 135 °C | cst | 986 | 1188 | 1657 | 1515 |
| Viscosity 165 °C | cst | 250 | 290 | 370 | 345 |

| ELASTOM. PROPS. | | | | | |
|---|---|---|---|---|---|
| Ductility 5°C | cm | 19 | 10 | 10 | 1 |
| Elast.recov. 10 °C | % | 67 | 65 | 60 | 50 |
| Def. E = 1000 % | Kg/cm² | 0,83 | 1,40 | 1,73 | 1,50 |
| Def. E = 2000 % | Kg/cm² | 0,63 | 1,13 | 1,27 | - |
| RM Bitumen | Kg/cm² | 2,65 | 3,70 | 3,84 | 3,78 |
| RM Polymer | Kg/cm² | 0,84 | 1,42 | 1,74 | 1,63 |
| Rupture elong. | E % | 3400 | 2850 | 2600 | 1400 |
| | Kg/cm² | 0,14 | 0,34 | 0,5 | 0,36 |
| RPM / RMB | | 0,32 | 0,38 | 0,45 | 0,43 |

## Claims

1. Process for the preparation of cross-linked hydrocarbonated polymeric binders, characterized in that it is performed by means of a two stage isothermic process, in which the quality of the product obtained in each stage is subjected to electronic control by "in situ" measurement of the viscosity, a fundamental property for the intended industrial application.

2. Process according to claim 1, characterized in that a first stage during which, under agitation and at a temperature ranging between 120 °C and 180 °, a polymeric concentrate constituted by the mixture of one hydrocarbonated solvent and a styrene-butadiene copolymer, is prepared; the solution of the polymer and consequently the homogeneization of the polymeric concentrate are controlled by "in situ" measurement of its viscosity by means of an adequate electronic system. During the first phase of the claimed process, these values have to be comprised between 400 cSt and 800 cSt measured at the solution temperature.

3. Process according to claim 1, characterized in that the cross-linked hydrocarbonated polymeric binder is obtained when the polymeric concentrate obtained in the first stage is mixed, in a second stage, with an asphaltene rich petroleum fraction, under agitation and at a temperature between 120 and 180 °C, and subsequently cross-links, within the same reactor, by the action of an adequate cross-linking system, similar to that used in the rubber vulcanization industry.

4. Process according to claims 1 and 3, characterized in that the duration of the cross-linking reaction or second stage is controlled by the use of an electronic system which determines the homogeneity of the cross-linked hydrocarbonated polymeric binder obtained by "in situ" measurement of the viscosity as fundamental property. These values must be comprised between 500 and 900 cSt measured at the reaction temperature.

5. Process according to claims 1, 2 and 3, characterized in that the appropriate selection of the components of the cross-linked hydrocarbonated polymeric binder permits the performance of the whole process to be carried out at a constant temperature, coinciding with that of the raw materials and the products.

6. Process according to claim 1, characterized in that the hydrocarbonated solvent utilized remains as part of the final product in its use as binder in asphaltic agglomerates as in construction sealants. Said solvent is characterized by having an aniline point between 60 and 80 °C, according to ASTM standard D-611 and a kinematic viscosity between 50 cSt and 70 cSt, according to ASTM standard D-445. Although its proportion in the final product depends upon the adjustment made in order to meet required specifications, usually this will lie between 15 and 45 pph of the final product.

7. Process according to claim 2, characterized in that the appropriate copolymers for the process related to this invention are sequential block polymers, even random, both linear and radial, which comprise conjugated styrenic and dienic groups , being butadiene the one used in this process. The fundamental characteristic which makes them useful for this process is their Mooney viscosity which is comprised between 45 and 65 (ML4, 100 °C). The styrene/butadiene ratio lies in all cases between 0.25 and 0.45 . Its proportion in the final product ranges between 2 and 6 pph.

8. Process according to claim 3, characterized in that the asphaltene rich petroleum fraction employed is obtained by the vacuum distillation of crude oil, and is defined by having an asphaltene content of over 7% as determined by the IP-143 method, and a flashpoint above 235 °C according to ASTM standard D-92/90. The proportion of the asphaltene rich petroleum fraction found in the final product is variable, depending on the characteristics of the final product to be obtained, although it is generally between 50 and 85 % in weight of the final product.

9. Process according to claims 1 and 3, characterized in that the properties of the cross-linked hydrocarbonated polymeric binder may be adjusted, if necessary, by the addition of a hydrocarbonated solvent characterized by claim 6, as well as by oxidation techniques used in the field of oxidized bitumens.

10. Use of the cross-linked hydrocarbonated polymeric binder obtained according to claims 1 and 3 or 1, 3 and 9 as a binder in the preparation of asphaltic agglomerates and as a sealant in construction joints.
